(19) **Europäisches Patentamt**

**European Patent Office**

**Office européen des brevets**

(11) **EP 1 455 801 B1**

(12) **EUROPÄISCHE PATENTSCHRIFT**

(45) Veröffentlichungstag und Bekanntmachung des Hinweises auf die Patenterteilung:
**09.03.2005 Patentblatt 2005/10**

(21) Anmeldenummer: **01990585.0**

(22) Anmeldetag: **21.12.2001**

(51) Int Cl.⁷: **A61K 31/7056**, A61K 9/16,
A61P 31/12

(86) Internationale Anmeldenummer:
**PCT/EP2001/015202**

(87) Internationale Veröffentlichungsnummer:
**WO 2003/053450 (03.07.2003 Gazette 2003/27)**

(54) **RIBAVIRIN-GRANULAT ZUR HERSTELLUNG VON FILMTABLETTEN**

RIBAVIRIN GRANULATE FOR PRODUCING COATED TABLETS

GRANULAT DE RIBAVIRINE POUR LA PRODUCTION DE COMPRIMES PELLICULES

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(43) Veröffentlichungstag der Anmeldung:
**15.09.2004 Patentblatt 2004/38**

(73) Patentinhaber: **Biopartners GmbH**
**6340 Baar (CH)**

(72) Erfinder:
• **SOBEL, Cornelius**
**55130 Mainz (DE)**
• **HUBER, Gerald**
**73655 Plüderhausen (DE)**

(74) Vertreter: **Krauss, Jan et al**
**Forrester & Boehmert,**
**Pettenkoferstrasse 20-22**
**80336 München (DE)**

(56) Entgegenhaltungen:
**EP-A- 0 795 324**          **WO-A-01/62229**
**US-A- 4 211 771**

**Beschreibung**

Die Erfindung betrifft ein Ribavirin-Granulat zur Herstellung von Ribavirin-enthaltenden Tabletten sowie deren Verwendung.

Der Freiname "Ribavirin" (1-β-D-Ribafuranosyl-1,2,4-triazol-3-carboxamid) bezeichnet ein Nukleosidanalogon, das als Virustatikum mit begrenzter Indikation zur Therapie am Menschen zugelassen ist. Aufgrund seiner chemisch modifizierten Ribose-Einheit weist Ribavirin das typische Merkmal eines Antimetaboliten bzw. seines Vorläufermoleküls auf. Beschrieben wurde eine Hemmung der Synthese von Guanosin-Nukleosiden durch Ribavirin, eine Hemmung der RNA-Polymerase und eine indirekte Hemmung der Proteinbiosynthese. Was allerdings dem genauen Wirkungsmechanismus von Ribavirin zugrunde liegt, ist noch immer ungeklärt.

Prinzipiell wirkt Ribavirin gegen ein breites Spektrum von Viren (z. B. Hepatitis, Influenza, Masern, Herpes, AIDS). Therapeutisch relevant sind gegenwärtig nur seine Aktivitäten gegen das *Respiratory-Syncytial*-Virus (RSV) und das Hepatitis C-Virus (HCV).

In Deutschland war Ribavirin zunächst ausschließlich zur Aerosoltherapie schwerster bronchopulmonaler Infektionen, verursacht durch RSV, zugelassen. Unter dem Handelsnamen vor allem jedoch bei Rückfall-Patienten mit chronischer Hepatitis C, die bereits früher auf Interferon α angesprochen hatten.

Die bisher einzige perorale Dareichungsform von Ribavirin, nämlich Rebetol®, sind Hartkapseln auf der Basis von Gelatine.

Aus phamazeutischer Sicht weisen Kapseln eine Reihe von Nachteilen auf. Die Genauigkeit, mit der der Wirkstoff dosiert werden kann, ist bei Kapseln geringer als beispielsweise bei Tabletten. Entsprechend beträgt die gesetzlich höchstzulässige Varianz der Masse je Dosiereinheit ± 7,5 % für Kapseln und ± 5 % für Tabletten. Vom Patienten ist die Kapsel möglichst intakt zu schlucken, was bei bestimmten Personen (Kindern, älteren Menschen, Schwerkranken) problematisch sein kann und u. U. die Behandlungsbereitschaft ("Compliance") beeinträchtigt. Die Wirkstoffreisetzung erfolgt dann mit dem raschen Zerfall der Kapsel im Magen. Eine zeitliche Steuerung der Freisetzung ist praktisch nicht möglich.

Die Verpackung von Arzneistoffen in Kapseln hat darüberhinaus auch praktische Nachteile. Eine Produktion unterschiedlicher Dosiereinheiten in homologer Reihe ist technisch wesentlich aufwendiger als z. B. bei Tabletten. Eine flexible Dosierung durch einfaches Zerteilen ist bei Kapseln ebenfalls nicht möglich. Eine weitere kritische Eigenschaft, die in letzter Zeit zunehmende Bedeutung erlangt hat, ist, daß Kapseln Bestandteile tierischen Ursprungs, wie etwa Gelatine, enthalten und damit die Gefahr bergen, z. B. den BSE-Erreger zu übertragen.

Im Tierexperiment wurde gezeigt, daß Ribavirin teratogen wirkt, insbesondere innerhalb der ersten sechs Wochen der Embryonalentwicklung. Dies hat u. a. zur Folge, daß weibliches Behandlungspersonal vor einer Exposition geschützt werden muß und weiterhin, daß die Beteiligung von Frauen an der Produktion Ribavirin-haltiger Arzneimittel auszuschließen ist. Beim Zusammenstecken der Kapseln ist es unvermeidbar, daß Ribavirin-Pulver an deren äußerer Oberfläche hängen bleibt. Anders als Tabletten können Gelatinekapseln nicht mit einer wäßrigen Lackierung, die geeignet ist, das Pulver fest einzubinden, überzogen werden. Nicht nur beim Verpacken der Kapseln, sondern auch bei der Verabreichung, z. B. durch eine Pflegekraft, ist die Wahrscheinlichkeit deshalb groß, mit Ribavirin in Kontakt zu kommmen. Schließlich sind die mehr oder weniger locker zusammengesteckten Kapseln mechanisch nicht sehr stabil, was ein zusätzliches Risiko in der Handhabung und vor allem auch beim Transport bedeuten kann.

Zusätzlich zu den allgemeinen Nachteilen einer Kapsel ist deshalb speziell im Fall von Ribavirin eine andere perorale Darreichungsform wünschenswert.

Der Erfindung liegt die Aufgabe zugrunde, Ribavirin in Darreichungsform einer Tablette, insbesondere einer Filmtablette, zur Verfügung zu stellen.

Erfindungsgemäß wird die Aufgabe dadurch gelöst, daß ein Verfahren zur Herstellung eines Ribavirin-enthaltenden Granulats verfügbar gemacht wird, umfassend:

- Herstellen einer Granulierlösung, umfassend Mischen von Bindemittel mit einem Isopropanol/Wasser oder Ethanol/Wasser-Gemisch,
- Einrühren der Granulierlösung in ein Gemisch von Ribavirin-Pulver mit einem hydrophilen oder quellbaren festen Hilfsstoff, und
- Sieben und Trocknen des so erhaltenen Granulats, dadurch gekennzeichnet, daß der Anteil von Isopropanol oder Ethanol am Alkohol/Wasser-Gemisch von 65 Gew-% bis 85 Gew-%, bevorzugterweise bei Verwendung von Ethanol von 75 Gew-% bis 85 Gew-% und bei der Verwendung von Isopropanol von 65 Gew-% bis 75 Gew-% beträgt.

In einem bevorzugten Verfahren ist das Bindemittel ausgewählt aus Cellulose und/oder Derivaten davon oder Stärke und/oder Derivaten davon. Besonders bevorzugt ist Polyvinylpyrrolidon, insbesondere Polyvidon mit einem Molekulargewicht von weniger als 90.000, wie z.B. K25.

In einem bevorzugten Verfahren ist der hydrophile oder quellbare feste Hilfsstoff ausgewählt aus mikrokristalliner Cellulose, Zuckeralkoholen, Stärke (z. B. Maisstärke) und/oder Derivaten davon oder Zuckern (z. B. Milchzucker) und/oder Derivaten davon.

In einem weiteren bevorzugten Verfahren wird das feuchte Granulat mit einem ≤ 5 mm, bevorzugt 2 mm, Sieb gesiebt. Dadurch wird das Granulat homogen, ist aber noch zu grob zur weiteren Verarbeitung.

In einem weiteren bevorzugten Verfahren wird das Granulat bis zu einer Restfeuchte (Meßtemperatur von 70°C) von weniger als 3% getrocknet.

In noch einem weiteren bevorzugten Verfahren wird das getrocknete Granulat abschließend mit einem ≤ 2 mm, bevorzugt 1 mm, Sieb gesiebt. Durch diese Behandlung wird der unerwünschte Staubanteil entscheidend minimiert und die Teilchengrößenverteilung für die nachfolgenden Prozeßschritte optimiert.

Besonders bevorzugt ist die Verwendung des Granulats zur Herstellung einer Ribavirin-enthaltenden Tablette, wobei das Verfahren zur Herstellung umfaßt:

- Mischen des Ribavirin-Granulats mit mikrokristalliner Cellulose, Sprengmittel und hochdispersem Siliziumdioxid und/oder Talkum und/oder Kalziumhydrogenphosphat,
- Hinzufügen von einem Schmiermittel, wie z. B. Magnesiumstearat, Fumarsäure, Adipinsäure oder PEG, und
- Tablettieren des Gemisches.

In einem bevorzugten Verfahren zur Herstellung einer Ribavirin-Tablette ist das Sprengmittel quervemetztes Polyvinylpyrrolidon, insbesondere Crospovidon oder ein anderer in Wasser quellender Hilfsstoff.

Ein besonders bevorzugtes Verfahren zur Herstellung einer Ribavirin-Tablette umfaßt das Aufbringen einer Filmbeschichtung.

In einem besonders bevorzugten Verfahren zur Herstellung einer Ribavirin-Tablette umfaßt die Filmbeschichtung Titandioxid oder ein anderes geeignetes Pigment; Isopropanol, Ethanol, Wasser oder Gemische von diesen; und einen Filmbildner, wie z. B. Hydroxypropylmethylcellulose, Hydroxypropylmethylcellulosephthalat, Ethylcellulose, Polyacrylate oder Schellack und Derivate davon.

Die oben angegebenen Hilfs- und Zusatzstoffe zur Herstellung des erfindungsgemäßen Granulats und der erfindungsgemäßen Tablette stellen bevorzugte Ausführungsformen dieser Stoffe dar. Es ist dem Fachmann auf diesem Gebiet leicht ersichtlich, daß diese Stoffe auch durch andere gleichwertige Stoffe ersetzt bzw. ergänzt werden können, ohne sich von dem generellen Konzept der vorliegenden Erfindung zu entfernen. Geeignete weitere Stoffe kann der Fachmann insbesondere den folgenden Standardwerken entnehmen: Pharmazeutische Technologie: hrsg. von Heinz Sucker, bearb. von H. Asche - 2., neu bearb. Aufl. - Stuttgart; New York: Thieme, 1991. - XXII, 801 S.; ISBN 3-13-395802-X; Pharmazeutische Technologie: Kurt H. Bauer; Karl-Heinz Frömming; Claus Führer. Unter Mitarb. von Engelbert Graf - 5., überarb. Aufl. - Stuttgart; Jena; Lübeck; Ulm: Fischer; Frankfurt [Main]: Govi-Verl., 1997. - XV, 472 S.; ISBN 3-437-25630-0, 3-7741-0638-X ; Ab 6. Aufl. u.d.T.: Lehrbuch der pharmazeutischen Technologie; Pharmazeutische Technologie: moderne Arzneiformen: Lehrbuch für Studierende der Pharmazie, Nachschlagewerk für Apotheker in Offizin, Krankenhaus und Forschung; 72 Tabellen / von Rainer H. Müller und Gesine E. Hildebrand. Mit Beitr. von: K. H. Bauer - 2., durchges. und erw. Aufl. - Stuttgart: WVG, Wiss. Verl.-Ges., 1998. - XVII, 471 S. : Ill.; (dt.); ISBN 3-8047-1549-4; Pharmazeutische Technologie: industrielle Herstellung und Entwicklung von Arzneimitteln; Ingfried Zimmermann. - Berlin; Heidelberg; New York; Barcelona; Budapest; Hon*: Springer, 1998. - XX, 644 S.; ISBN 3-540-63944-6; Pharmazeutische Technologie: für Studium und Beruf; von Rudolf Voigt. - 9., völlig überarb. Aufl. / bearb. von Alfred Fahr. - Stuttgart: Dt. Apotheker-Verl., 2000. - XXXII, 687 S.; (Wissen & Praxis); ISBN 3-7692-2649-6; bis zur 8. Aufl. erschienen im Verlag Ullstein Mosby Wiesbaden; Literaturverzeichnis S. [649] - 651; Arzneiformenlehre: ein Lehrbuch der Galenik für Theorie und Praxis; mit 44 Tabellen / von Ursula Schöffling. - 3., völlig neu bearb. und erw. Aufl. - Stuttgart: Dt. Apotheker-Verl., 1998. - 464 S.; ISBN 3-7692-2254-7; Propädeutikum der Arzneiformenlehre / Claus-Dieter Herzfeldt. - 2. Aufl. - Berlin; Heidelberg: Springer, 2000. - XVI, 249 S.; Galenik / Claus-Dieter Herzfeldt; 1; (Springer-Lehrbuch); ISBN 3-540-65265-5; Grundlagen der Arzneiformenlehre Claus-Dieter Herzfeldt ... (Hrsg.). - Berlin; Heidelberg: Springer, 1999. - XV, 618 S.; Galenik / Claus-Dieter Herzfeldt; 2; (Springer-Lehrbuch); ISBN 3-540-65291-4; Propädeutische Arzneiformenlehre: Einführung in die Arzneiformenherstellung in der Apotheke; von Engelbert Graf u. Christian Beyer. - 3., völlig neu bearb. Aufl. von Christian Beyer. - Stuttgart: Wissenschaftliche Verlagsgesellschaft, 1993. - 206 S.; ISBN 3-8047-1267-3; Physikalische Pharmazie: pharmazeutisch angewandte physikalisch-chemische Grundlagen; Martin; Swarbrick; Cammarata. Hrsg. u. vollst. überarb. von H. Stricker. - 3., völlig neu bearb. u. erw. Aufl. - Stuttgart: Wiss. Verl. -Ges., 1987. - XVI, 587 S.; ISBN 3-8047-0893-5; Original: Physical pharmacy; und Lexikon der Hilfsstoffe für Pharmazie, Kosmetik und angrenzende Gebiete / Herbert P. Fiedler. - Aulendorf: Ed. Cantor; Der pharmazeutische Betrieb; Bd. 9; ISBN 3-87193-101-2, 3-87193-173-X Lexikon der Hilfsstoffe für Pharmazie, Kosmetik und angrenzende Gebiete / Herbert P. Fiedler); - 3., überarb. und erw. Aufl. - 1989.

Besonders bevorzugt ist eine Ribavirin-enthaltende Tablette, die nach dem erfindungsgemäßen Verfahren hergestellt ist.

Besonders bevorzugt ist die therapeutische Verwendung der Ribavirin-Tablette zur Behandlung von indizierten Erkrankungen, insbesondere viralen Erkrankungen wie HCV. Weiterhin kann die Therapie in Kombination mit einem Zytokin, insbesondere einem Interferon, wie z.B. Interferon alpha und Derivaten davon erfolgen.

Als erfindungswesentlich für Struktur und Fließfähigkeit des Granuats stellte sich der Isopropanol/ Ethanol-Anteil der Granulierlösung heraus, in der das Ribarin-Pulver bei der Herstellung aufgenommen wird. Ist er zu hoch, wird das Granulat mechanisch instabil und schlecht fließfähig. Umgekehrt ist bei einem hohen Wasser-Anteil das Granulat zu hart und spöde, um verpreßt zu werden. In beiden Fällen steigt vor allem der unerwünschte Staubanteil stark an. Überraschenderweise erwies sich ein ungefähr 80%iger Anteil von Ethanol am Ethanol/Wasser-Gemisch oder ein ungefähr 70%iger Anteil von Isopropanol am Isopropanol/Wasser-Gemisch als optimal.

Die guten Fließeigenschaften des Granulats tragen wesentlich dazu bei, die vorgesehene Ribavirin-Dosis je Tablette möglichst exakt einzuhalten, was im Hinblick auf die geforderte Arzneimittelsicherheit von erheblicher Bedeutung ist. Die mittlere Wirkstoffmenge in der Arzneiform variiert um etwa ± 3%, womit die Abweichung niedriger ist, als gesetzlich zulässig (± 5%), und deutlich niedriger, als bei Kapseln gesetzlich zulässig. Auch bleibt aufgrund der guten Fließfähigkeit der Anteil an produziertem Ausschuß gering. Die Produktion unterschiedlicher Dosiereinheiten in homologer Reihe ist auch bei definierter Zusammensetzung des Granulats ohne weiteres möglich, indem das Gewicht der einzelnen Tablette entsprechend verändert wird.

Ein wesentlicher Vorteil der Ribavirin-Tablette besteht weiterhin darin, daß sie mit einem wasserlöslichen Film beschichtet werden kann. Durch einen solchen Film wird der noch eventuell vorhandene Ribavirin-Staub gebunden und die Tablette versiegelt. Personen und Umwelt werden auf diese Weise vor dem teratogen wirkenden Ribavirin besser geschützt. Weiterhin wird mittels der Filmtablette eine gewisse zeitliche Steuerung der Ribavirin-Freisetzung im Magen möglich.

Im folgenden werden die vorteilhaften Eigenschaften der Erfindung anhand zweier Darstellungen näher ausgeführt:

Figur 1:  Härte der Ribavirin-Tabletten als Ergebnis der Preßkraft beim Tablettieren

Figur 1a: Füllmenge-Härte-Diagramm
Figur 1b: Preßkraft-Härte-Diagramm

Figur 2:  Freisetzungskinetik von Ribavirin: Vergleich der Ribavirin-Tabletten mit Hartgelatinekapseln

Figur 2a: Vergleich der Freisetzungskinetik von Ribavirin (200 mg) aus Hartgelatinekapseln (Rebetol®-Muster) und Filmtabletten bei pH 5,5 (Mund) (Phosphatpuffer). Angegeben sind die Mittelwerte aus drei Experimenten.
Figur 2b: Vergleich der Freisetzungskinetik von Ribavirin (200 mg) aus Hartgelatinekapseln (Rebetol®-Muster) und Filmtabletten bei pH 1,0 (Magen) (0,1 N HCl). Angegeben sind die Mittelwerte aus sechs Experimenten.

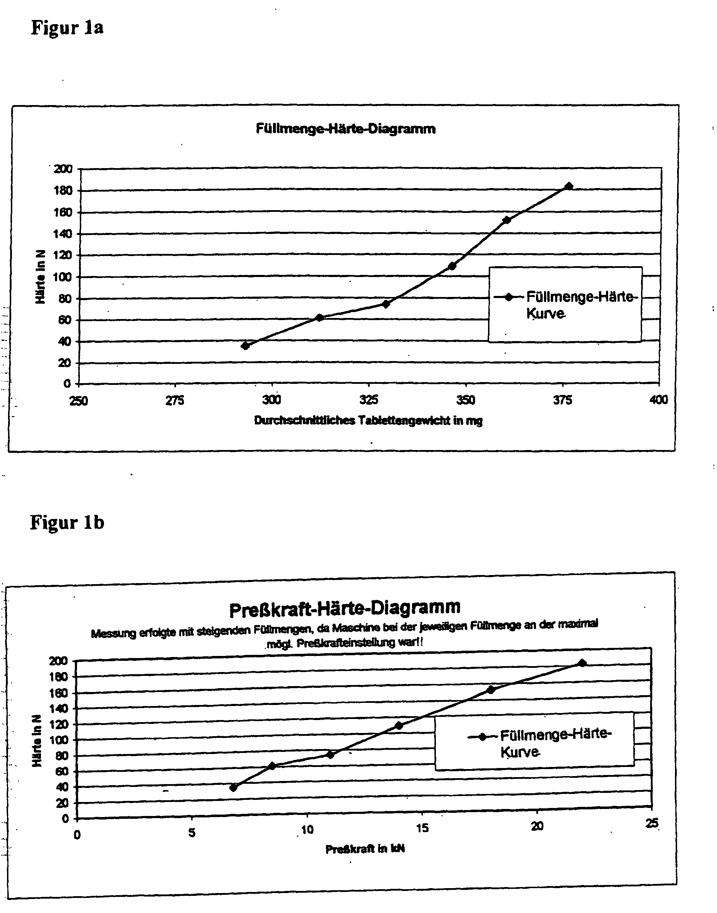

Figur 1 stellt den Zusammenhang zwischen der Preßkraft, die beim Tablettieren auf das Granulat wirkt und der Härte der resultierenden Ribavin-Tabletten bei einem Durchmesser von 10,4 mm dar.

Die beim Verpressen der Ribavirin-Endmischung aufgewendete Kraft ist bei einer Füllmenge von 300 mg je Dosiereinheit maximal und kann technisch bedingt nicht weiter gesteigert werden. Durch Erhöhung der Füllmenge wurde deshalb eine Erhöhung der Preßkraft simuliert (Fig. 1a). Es zeigte sich, daß die erzielte Tablettenhärte in Abhängigkeit vom durchschnittlichen Tablettengewicht zunahm, und zwar von 35 N bei 293 mg auf 152 N bei 360 mg. Mit 376 mg Tablettengewicht wurde schließlich ein Grenzwert erreicht, oberhalb dessen gute Tablettiereigenschaften nicht mehr gewährleistet waren.

Wird die Tablettenhärte in Bezug zur Preßkraft gesetzt (Fig. 1b), entsprechen die erzielten Härten einer Preßkraft zwischen 6,8 kN (bei 35 N Härte) und 18 kN (bei 152 N Härte).

Aufgrund der vorteilhaften Struktur des Granulats genügt eine Preßkraft mittlerer Größe (6,8-8,5 kN), um Tabletten mit befriedigender Härte (35-61 N) herzustellen (siehe Fig. 1). Da nur mäßig Wärme erzeugt wird, ist der Wirkstoff entsprechend wenig thermischer Belastung ausgesetzt.

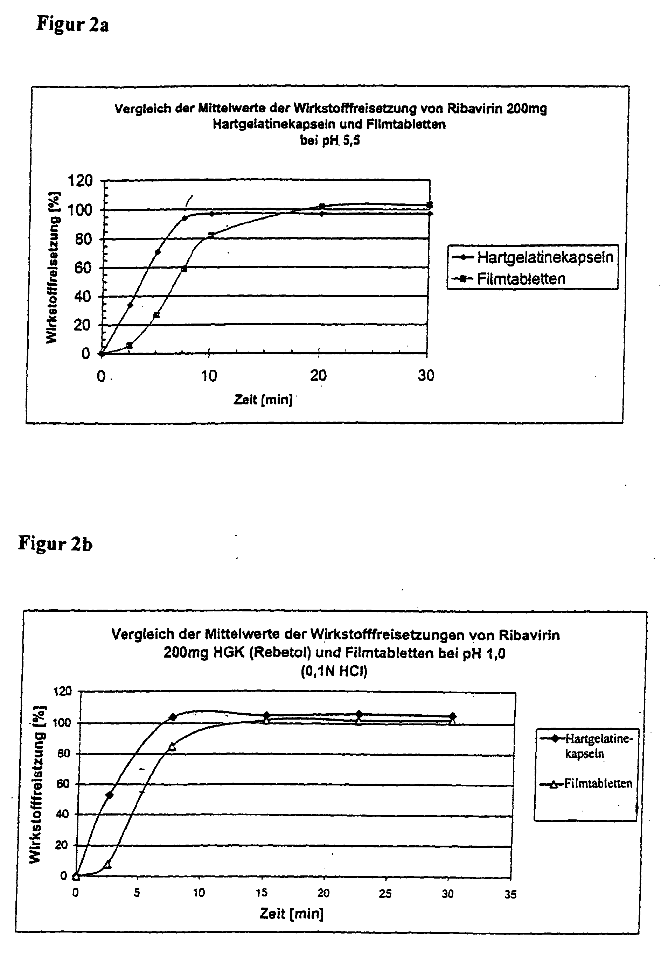

Figur 2 zeigt die Freisetzungseigenschaften der Tablette dieser Erfindung *in vitro.* Aufgrund der leicht verzögerten Freisetzung wird die Arzneimittelsicherheit der Tablette der vorliegenden Erfindung zusätzlich im Vergleich zu den bisherigen Kapseln gesteigert, da zwischen Schlucken (Tablette im Mund) und der Freisetzung (Tablette im Magen) eine gewisse Zeit vergeht. Dieser Effekt ist zudem dosisunabhängig und läßt sich auch bei kleinen Tablettengrößen erzielen. Aus Hartgelatinekapseln hingegen wird Ribavirin sowohl unter schwach sauren Bedingungen (Fig. 2a), wie sie in der Mundhöhle herrschen, als auch bei einem dem Magensaft entsprechenden pH-Wert (Fig. 2b) unverzüglich freigesetzt, was für die Arzneimittelsicherheit bedenklich sein kann.

Im folgenden werden zwei Ausführungsbeispiele der Erfindung dargestellt:

Beispiel 1: In einer Ausführung enthält eine Tablette 200 mg ± 3% Ribavirin sowie Zusatzstoffe gemäß der Rezeptur:

| Ribavirin | 200,00 mg |
| Polyvidon K25 | 16,00 mg |
| Mikrokristalline Cellulose | 77,00 mg |
| Crospovidon | 3,50 mg |
| Siliciumoxid | 2,00 mg |
| Magnesiumstearat | 1,50 mg |
| insgesamt | 300,00 mg |

Beispiel 2: In einer besonders bevorzugten Ausführung ist die Tablette der oben beschriebenen Rezeptur zusätzlich mit einem wasserlöslichen Film beschichtet ("Lackierung", "Coating"), zusammengesetzt aus:

| Hydroxypropylmethylcellulose | 4,00 mg |
|---|---|
| Titandioxid | 2,00 mg |
| | 6,00 mg |

oder alternativ zusammengesetzt aus:

| Hydroxypropylmethylcellulose | 4,00 mg |
|---|---|
| Titandioxid | 2,00 mg |
| Polyethylenglykol 6000 (Macrogol 6000) | 1,00 mg |
| | 7,00 mg |

Beispielhaft wird nachfolgend die Herstellung einer Charge von Ribavirin-Tabletten im Produktionsmaßstab beschrieben:

Alle im Verfahren eingesetzten Substanzen erfüllen die Anforderungen des Arzneimittelgesetzes. Entsprechendes gilt für die verwendeten Gerätschaften.

1. Herstellung von Ribavirin-Granulat

1.1. Vormischung I (Granulierlösung):

Die Granulierlösung besteht aus Polyvinylpyrrolidon (Polyvidon K25, Kollidon 25, bezogen von BASF, Ludwigshafen) (2,00 kg), das in einer Mischung aus Ethanol 96% (9,02 kg) und Wasser (3,25 kg) durch Unterrühren vollständig gelöst wird (Gesamtgewicht: 14,27 kg).

1.2. Vormischung II (Granulat):

Ribavirin (36,3 kg) und mikrokristalline Cellulose (Avicel PH101, bezogen von Lehmann & Voss, Hamburg) (6 kg) werden zunächst gemischt und anschließend durch Zugabe der Granulierlösung (14,27 kg; Eintragzeit ungefähr 10 Minuten) zu einem homogenen Granulat verarbeitet. Das Granulat wird durch ein 2,00 mm Sieb Frewitt feucht gesiebt und bei 60°C ungefähr 3,5 Stunden bis zu einer Restfeuchte von weniger als 3% (Meßtemperatur von 70°C) getrocknet. Schließlich wird das trockene Granulat durch ein 1,00 mm Sieb Frewitt gesiebt, um eine gleichmäßige und staubarme Granulatstruktur zu erreichen.

2. Herstellung der Ribavirin-Endmischung

Zu einer Mischung aus mikrokristalline Cellulose (2,662 kg), Polyvinylpyrrolidon (Crospovidon, Kollidon CL, bezogen von BASF) (0,212 kg), hochdispersem Siliciumdioxid (Aerosil 200, bezogen von Degussa) (0,121 kg) und Polyvidon K 25 (0,303 kg) wird Ribavirin-Granulat (14,767 kg) gemischt. Zu dieser Mischung wird Magnesiumstearat (0,091 kg) gegeben und erneut gemischt.

3. Tablettierung

Die Ribavirin-Endmischung wird in Portionen von ungefähr 300 mg (entsprechend 200 mg Ribavirin) maschinell zu bikonvexen Tabletten von ungefähr 4,5-5,5 mm Höhe und einem Druchmesser von ungefähr 10 ± 12 mm, bevorzugt 9,0-9,7 mm verpreßt.

4. Filmbeschichtung

Die Beschichtung der Tablettenkerne erfolgt durch Sprühauftrag einer Mischung aus Titandioxid (0,196 kg), supendiert in Wasser (0,5 kg), und Hydroxypropylmethylcellulose (Pharmacoat 606, bezogen von Synthapharm) (0,392 kg), die zuvor vollständig in Wasser (2,5 kg) gelöst wurde.

**Patentansprüche**

1. Verfahren zur Herstellung eines Ribavirin enthaltenden Granulats, umfassend

   - Herstellen einer Granulierlösung, umfassend Mischen von Bindemittel mit einem Isopropanol/Wasser oder Ethanol/Wasser-Gemisch,
   - Einrühren der Granulierlösung in ein Gemisch von Ribavirin-Pulver mit einem hydrophilen oder quellbaren festen Hilfsstoff, und
   - Sieben und Trocknen des so erhaltenen Granulats, **dadurch gekennzeichnet, daß** der Anteil von Isopropanol oder Ethanol am Alkohol/Wasser-Gemisch von 65 Gew-% bis 85 Gew-% beträgt.

2. Verfahren zur Herstellung eines Ribavirin enthaltenden Granulats nach Anspruch 1, **dadurch gekennzeichnet, daß** der Anteil am Alkohol/Wasser-Gemisch bei Verwendung von Ethanol von 75 Gew-% bis 85 Gew-% und bei der Verwendung von Isopropanol von 65 Gew-% bis 75 Gew-% beträgt.

3. Verfahren zur Herstellung eines Ribavirin enthaltenden Granulats nach Anspruch 1 oder 2, **dadurch gekennzeichnet, daß** das Bindemittel ausgewählt ist aus Cellulose und/oder Derivaten davon oder Stärke und/oder Derivaten davon, insbesondere Polyvinylpyrrolidon, insbesondere Polyvidon mit einem Molekulargewicht von weniger als 90.000, wie z. B. K25.

4. Verfahren zur Herstellung eines Ribavirin enthaltenden Granulats nach einem der Ansprüche 1 bis 3, **dadurch gekennzeichnet, daß** der hydrophile oder quellbare feste Hilfsstoff aus mikrokristalliner Cellulose, Zuckeralkoholen, Stärke und/oder Derivaten davon oder Zuckern (z. B. Milchzucker) und/ oder Derivaten davon ausgewählt ist.

5.   Verfahren zur Herstellung eines Ribavirin enthaltenden Granulats nach einem der Ansprüche 1 bis 4, **dadurch gekennzeichnet, daß** das feuchte Granulat mit einem ≤ 5 mm, bevorzugt 2 mm, Sieb gesiebt wird.

6.   Verfahren zur Herstellung eines Ribavirin enthaltenden Granulats nach einem der Ansprüche 1 bis 5, **dadurch gekennzeichnet, daß** das Granulat bis zu einer Restfeuchte von weniger als 3% (Meßtemperatur von 70°C) getrocknet wird.

7.   Verfahren zur Herstellung eines Ribavirin enthaltenden Granulats nach einem der Ansprüche 1 bis 6, weiterhin umfassend ein abschließendes Sieben des getrockneten Granulats mit einem ≤ 2 mm, bevorzugt 1 mm, Sieb.

8.   Verwendung eines Ribavirin-Granulats gemäß einem der voranstehenden Ansprüche zur Herstellung einer Ribavirin-Tablette.

9.   Verfahren zur Herstellung einer Ribavirin-enthaltenden Tablette, umfassend

- Mischen des Ribavirin-Granulats, hergestellt nach einem der Ansprüche 1 bis 7, mit mikrokristalliner Cellulose, Sprengmittel und hochdispersem Siliziumdioxid und/oder Talkum und/ oder Kalziumhydrogenphosphat,
- Hinzufügen von einem Schmiermittel, wie z.B. Magnesiumstearat, Fumarsäure, Adipinsäure oder PEG, und
- Tablettieren des Gemisches.

10.   Verfahren zur Herstellung einer Ribavirin-enthaltenden Tablette nach Anspruch 9, **dadurch gekennzeichnet, daß** das Sprengmittel quervenetztes Polyvinylpyrrolidon, insbesondere Crospovidon, ist.

11.   Verfahren zur Herstellung einer Ribavirin-enthaltenden Tablette nach Anspruch 9 oder 10, weiterhin umfassend das Aufbringen einer Filmbeschichtung,

12.   Verfahren zur Herstellung einer Ribavirin-enthaltenden Tablette nach Anspruch 11, **dadurch gekennzeichnet, daß** die Filmbeschichtung Titandioxid oder ein anderes geeignetes Pigment; Isopropanol, Ethanol, Wasser oder Gemische davon; und einen Filmbildner, wie z.B. Hydroxypropylmethylcellulose, Hydroxypropylmethylcellulosephthalat, Ethylcellulose, Polyacrylate oder Schellack und Derivate davon, umfaßt.

13.   Ribavirin-enthaltende Tablette, hergestellt nach einem der Ansprüche 9 bis 12.

14.   Verwendung einer Ribavirin-enthaltende Tablette nach Anspruch 13 zur Herstellung eines Medikaments zur Behandlung von indizierten Erkrankungen, insbesondere viralen Erkrankungen wie HCV.

15.   Verwendung einer Ribavirin-enthaltenden Tablette nach Anspruch 14 in Kombination mit einem Zytokin, insbesondere einem Interferon, z.B. Interferon alpha und/oder seinen Derivaten.

## Claims

1.   Method for the production of a ribavirin-containing granulate, comprising

- producing a granulate solution, comprising mixing of a binding agent with an isopropanol/water- or ethanol/water-mixture,
- stirring of said granulate solution into a mixture of ribavirin-powder with a hydrophilic or swellable solid adjuvant, and
- sieving and drying the granulate thus obtained, **characterised in that** the portion of the isopropanol or ethanol of the alcohol/water-mixture is from 65% to 85% by weight.

2.   Method for the production of a ribavirin-containing granulate according to claim 1, **characterised in that** the portion of the alcohol/water-mixture is from 75% to 85% by weight when using ethanol and from 65% to 75% by weight when using isopropanol.

3.   Method for the production of a ribavirin-containing granulate according to claim 1 or claim 2, **characterised in that** the binding agent is selected from cellulose and/or derivatives thereof or starch and/ or derivatives thereof, in particular polyvinylpyrrolidone, in particular polyvidone with a molecular weight of less than 90.000, such as K25.

4.   Method for the production of a ribavirin-containing granulate according to one of claims 1 to 3, **characterised in that** the hydrophilic or swellable solid adjuvant is selected from microcrystalline cellulose, sugar alcohols, starch and/or derivatives thereof or sugars (e.g. milk sugar) and/or derivatives thereof.

5.   Method for the production of a ribavirin-containing granulate according to one of claims 1 to 4, **characterised in that** the humid granulate is sieved with a sieve of ≤ 5 mm, preferably 2 mm.

**6.** Method for the production of a ribavirin-containing granulate according to one of claims 1 to 5, **characterised in that** the granulate is dried to residual humidity of less than 3% (measuring temperature of 70°C).

**7.** Method for the production of a ribavirin-containing granulate according to one of claims 1 to 6, further comprising a final sieving of the dried granulate with a sieve of ≤ 2 mm, preferably 1 mm.

**8.** Use of a ribavirin-granulate according to one of the preceding claims for the production of a ribavirin-tablet.

**9.** Method for the production of a ribavirin-containing tablet, comprising

- mixing of the ribavirin-granulate produced according to any of claims 1 to 7 with microcrystalline cellulose, blasting agent and highly disperse siliciumdioxide and/or talcum and/or calciumhydrogen phosphate,
- adding a lubricant, such as magnesium stearate, fumaric acids, adipinic acid or PEG, and
- tabletting of said mixture.

**10.** Method for the production of a ribavirin-containing tablet according to claim 9, **characterised in that** the blasting agent is a cross-linked polyvinylpyrrolidone, in particular cropovidone.

**11.** Method for the production of a ribavirin-containing tablet according to claim 9 or 10, further comprising the application of a film coating.

**12.** Method for the production of a ribavirin-containing tablet according to claim 11, **characterised in that** the film coating comprises titanium dioxide or another suitable pigment; isopropanol, ethanol, water or mixtures thereof; and a film-forming agent, such as hydroxypropylmethyl cellulose, hydroxypropylmethyl cellulose phthalate, ethyl cellulose, polyacrylates or shellac and derivatives thereof.

**13.** Ribavirin-containing tablet, produced according to one of claims 9 to 12.

**14.** Use of a ribavirin-containing tablet according to claim 13 for the production of a medicament for the treatment of indicated diseases, in particular viral diseases, like HCV.

**15.** Use of a ribavirin-containing tablet according to claim 14 in combination with a cytokine, in particular an interferon, e.g. interferon $\alpha$ and/or its derivatives.

**Revendications**

**1.** Procédé de fabrication d'un granulé contenant de la ribavirine, comprenant :

- la fabrication d'une solution de granulation, comprenant le mélange du liant avec un mélange isopropanol/eau ou éthanol/eau,
- l'agitation de la solution de granulation dans un mélange de poudre de ribavirine avec un auxiliaire solide hydrophile et gonflable, et
- le tamisage et le séchage du granulé ainsi obtenu, **caractérisé en ce que** la proportion de l'isopropanol ou de l'éthanol par rapport au mélange alcool/eau est de 65 % en poids à 85 % en poids.

**2.** Procédé de fabrication d'un granulé contenant de la ribavirine selon la revendication 1, **caractérisé en ce que** la proportion par rapport au mélange alcool/eau en utilisant de l'éthanol est de 75 % en poids à 85 % en poids et en utilisant de l'isopropanol de 65 % en poids à 75 % en poids.

**3.** Procédé de fabrication d'un granulé contenant de la ribavirine selon la revendication 1 ou 2, **caractérisé en ce que** le liant est choisi parmi la cellulose et/ou ses dérivés ou l'amidon et/ou ses dérivés, en particulier la polyvinylpyrrolidone, en particulier la polyvidone avec un poids moléculaire de moins de 90 000, comme par exemple K25.

**4.** Procédé de fabrication d'un granulé contenant de la ribavirine selon l'une des revendications 1 à 3, **caractérisé en ce que** l'auxiliaire solide hydrophile ou gonflable est choisi parmi la cellulose micro-cristalline, les alcools de sucre, l'amidon et/ou ses dérivés ou les sucres (par exemple lactose) et/ou ses dérivés.

**5.** Procédé de fabrication d'un granulé contenant de la ribavirine selon l'une des revendications 1 à 4, **caractérisé en ce que** le granulé humide est tamisé avec un tamis ≤ 5 mm, de préférence 2 mm.

**6.** Procédé de fabrication d'un granulé contenant de la ribavirine selon l'une des revendications 1 à 5, **caractérisé en ce que** le granulé est séché jusqu'à une humidité résiduelle de moins de 3 % (température de mesure de 70°C).

**7.** Procédé de fabrication d'un granulé contenant de la ribavirine selon l'une des revendications 1 à 6, comprenant en outre un tamisage final du granulé séché avec un tamis ≤ 2 mm, de préférence 1 mm.

**8.** Utilisation d'un granulé de ribavirine selon une des revendications précédentes pour la fabrication d'un comprimé de ribavirine.

9. Procédé de fabrication d'un comprimé contenant de la ribavirine, comprenant :

   - le mélange du granulé de ribavirine, fabriqué selon une des revendications 1 à 7, avec de la cellulose microcristalline, un agent d'éclatement et du dioxyde de silicium hautement dispersé et/ou du talc et/ou de l'hydrogénophosphate de calcium,
   - l'ajout d'un agent lubrifiant, comme par exemple le stéarate de magnésium, l'acide fumarique, l'acide adipique ou PEG, et
   - la compression du mélange.

10. Procédé de fabrication d'un comprimé contenant de la ribavirine selon la revendication 9, **caractérisé en ce que** l'agent d'éclatement est une polyvinylpyrrolidone à réticulation croisée, en particulier la crospovidone.

11. Procédé de fabrication d'un comprimé contenant de la ribavirine selon la revendication 9 ou 10, comprenant en outre le dépôt d'un film revêtement.

12. Procédé de fabrication d'un comprimé contenant de la ribavirine selon la revendication 11, **caractérisé en ce que** le film revêtement contient du dioxyde de titane ou un autre pigment approprié ; de l'isopropanol, de l'éthanol, de l'eau ou des mélanges de ceux-ci ; et un filmogène, comme par exemple l'hydroxypropylméthylcellulose, le phtalate d'hydroxypropylméthylcellulose, l'éthylcellulose, les polyacrylates ou le schellac et des dérivés de ceux-ci.

13. Comprimé contenant de la ribavirine, fabriqué selon l'une des revendications 9 à 12.

14. Utilisation d'un comprimé contenant de la ribavirine selon la revendication 13 pour la préparation d'un médicament pour le traitement des maladies induites, en particulier des maladies virales telles que le VHC (virus de l'hépatite C).

15. Utilisation d'un comprimé contenant de la ribavirine selon la revendication 14 en combinaison avec un cytokine, en particulier un interféron, par exemple interféron alpha et/ou ses dérivés.

## Figur 1a

Füllmenge-Härte-Diagramm

## Figur 1b

Preßkraft-Härte-Diagramm

Messung erfolgte mit steigenden Füllmengen, da Maschine bei der jeweiligen Füllmenge an der maximal mögl. Preßkrafteinstellung war!!

## Figur 2a

Vergleich der Mittelwerte der Wirkstofffreisetzung von Ribavirin 200mg Hartgelatinekapseln und Filmtabletten bei pH 5,5

## Figur 2b

Vergleich der Mittelwerte der Wirkstofffreisetzungen von Ribavirin 200mg HGK (Rebetol) und Filmtabletten bei pH 1,0 (0,1N HCl)